# EUROPÄISCHE PATENTANMELDUNG

(11) **EP 0 606 897 A2**
(43) Veröffentlichungstag der Anmeldung: **20.07.1994**
(21) Anmeldenummer: 94100364.2
(22) Anmeldetag: 12.01.1994
(51) Int. Cl.: A61N 5/06

(54) **UV-Licht-Filter**

(30) Priorität: 14.01.1993 DE 4300719
(71) Anmelder: IMAB-STIFTUNG, FL-9496 Balzers (LI)
(72) Erfinder: Brück, Gernot K., D-50858 Köln (DE)
(74) Vertreter: Lippert, Hans-Joachim, Dipl.-Ing.

(57) **Zusammenfassung**

Bei eisendotierten Quecksilberhochdruckstrahlern zur Erzeugung von UV-Licht, wie sie beispielsweise in Bräunungsgeräten Verwendung finden, ist es zur Absorption von unerwünschten Strahlungsarten erforderlich, der Strahlungsquelle einen Filter nachzuschalten. Bei den verwendeten Strahlungsquellen ist im Bereich des bräunungswirksamen UVB-Lichtes lediglich die Strahlung mit einer Wellenlänge von 313 nm ausgeprägt, so daß zur Einstellung der Bräunungsgeräte bzw. zur Eignung des Glasfilters zum Einsatz in derartigen Geräten die Kenntnis der Transmissionsrate für diese Strahlung wichtig ist. Es wird erfindungsgemäß daher vorgeschlagen, die Transmissionsrate jedes Einzelfilters für die 313 nm-Strahlung zu bestimmen und den Filter mit diesem Wert zu kennzeichnen, so daß die Bräunungsgeräte bei Kenntnis dieses Wertes sehr genau bezüglich der die Bräunung beeinflussenden Parameter eingestellt werden können. Zur Messung der Transmissionsrate wird vorgeschlagen, die 313 nm-Strahlung mittels eines geeigneten Emissionsmeßgerätes mit und ohne Filter zu messen und anschließend die Meßwerte miteinander zu vergleichen.

## Beschreibung

Die Erfindung betrifft eine Strahlenquelle zur Erzeugung einer insbesondere für kosmetische und/oder medizinische Zwecke verwendbaren Strahlung, mit einem nachgeschalteten Filter zur Absorption oder Abschwächung ungeeigneter oder unerwünschter Strahlung.

Darüber hinaus betrifft die Erfindung ein Verfahren zur Bestimmung der Transmissionsrate eines Filters für eine solche Strahlenquelle zur Festlegung der Bräunungswirkung des Strahlenquelle-Filter-Systems bzw. ein Bräunungsverfahren für Personen unter Verwendung einer UV-Licht-erzeugenden Strahlungsquelle, der ein Filter zur Absorption oder Abschwächung ungeeigneter oder unerwünschter Strahlung nachgeschaltet ist.

Die Erzeugung von Strahlung für den kosmetischen oder medizinischen Bereich erfolgt nach dem Stand der Technik üblicherweise mit Hilfe von Quecksilberhochdrucklampen, denen ein geeigneter Filter nachgeschaltet ist. Der Einsatz eines Filters muß deshalb zwingend erfolgen, weil die Quecksilberhochdruckstrahler typischerweise ein sehr breitbandiges Licht emittieren, und zwar vom hochenergiereichen Ultraviolett (UVC) bis zum langwelligen Infrarot (IR). Je nach Einsatzart sind aber nur sehr bestimmte Bereiche dieser Strahlung von Interesse, wohingegen andere Strahlungsbereiche möglichst vollständig weggefiltert werden müssen. Beispielsweise dürfen hochenergiereiche UVC-Strahlen keineswegs zur Körperbestrahlung eingesetzt werden, da sie aufgrund ihrer Strahlungswirkung lebende organische Substanz abtöten oder in ihrer Zusammensetzung verändern können.

Die Quecksilberhochdrucklampen können nur in sehr eingeschränktem Maße zur Emission bevorzugter Strahlungsarten abgeändert werden. Dies wird beispielsweise durch die Dotierung der Strahler mit bestimmten Metallen erreicht. Strahlern zur Erzeugung von bräunenden UV-Strahlen wird beispielsweise Eisen zugesetzt, da dadurch im hier interessierenden UV-Bereich mehr Emissionslinien besetzt sind, d.h. in dem gewünschten Bereich wird besonders viel Strahlung emittiert. Dies ändert jedoch nichts an der Tatsache, daß auch bei diesen Strahlern viel unerwünschtes bzw. ungeeignetes Licht erzeugt wird.

Bei der Einteilung von ultravioletter Strahlung wird die sogenannte UVC-Strahlung mit einer Wellenlänge bis 280 nm, die UVB-Strahlung mit einer Wellenlänge bis 315 nm und die UVA-Strahlung mit einer Wellenlänge bis 380 nm unterschieden. Für den kosmetischen Einsatz, also beispielsweise für Bräunungszwecke, ist nur das unsichtbare UVA-Licht und in geringem Maße auch das ebenfalls unsichtbare UVB-Licht von Nutzen. Jedes UVC-Licht und große Teile des UVB-Lichtes müssen in jedem Fall vom Benutzer eines Bräunungsgerätes ferngehalten werden. Auch das langwellige Infrarotlicht ist unerwünscht, da dieses als Hitzestrahlung unter Umständen bis zur Schmerzempfindung führen kann.

Neben den beschriebenen schädlichen Effekten durch die unerwünschten Strahlungsarten kann aber auch die gleißende Helligkeit als störend empfunden werden, da deren Intensität so stark sein kann, daß die Helligkeit selbst durch die geschlossenen Augenlider dringt. Hierdurch wird das Wohlbefinden des Benutzers gleichfalls stark eingeschränkt.

Aufgrund der geschilderten Problematik ergibt sich für die Hersteller von Bräunungsgeräten die Forderung, Filter für die Quecksilberhochdruckstrahler einzusetzen, die schädliches oder ungeeignetes Licht absorbieren und lediglich das in diesem Falle erwünschte UV-Licht in einem Bereich zwischen 300 bis 340 nm transmittieren. Zum Einsatz kommen bisher gefärbte Glasfilter, die diese Aufgabe jedoch nur sehr unzureichend erfüllen. Häufig wird das bräunungswirksame Licht durch den Filter selber oder einen nachgeschalteten Klarfilter absorbiert. Dieses findet seinen Grund in der Tatsache, daß sich bei der Produktion derartiger Filterscheiben große Unterschiede in ihren Transmissionsverhalten ergeben, so daß die Filter bzw. die Filterkombination so eingestellt wird, daß jedes UVB-Licht absorbiert wird. Hierdurch wird bei den Bräunungsgeräten zwar die Sicherheit erreicht, daß kein Erythem (Hautrötung) entsteht, auf der anderen Seite wird allerdings auch keine anhaltende Bräunung erzielt.

Die Folgen derartiger Filter sind den Benutzern von Sonnenbänken bekannt. Bei der Verwendung von Quecksilberhochdruckstrahlern wird nur eine kurz anhaltende grau-schwarze Bräune erzielt und dies auch nur bei schon vorgebräunten Personen.

Bei den zur Bräunung verwendeten eisendotierten Quecksilberstrahlern existieren im Bereich zwischen 315 nm und 340 nm nur wenige schwache Strahlungslinien. Im Bereich des UVB-Lichtes, also zwischen 280 und 315 nm, existiert nur das typische Quecksilberlinientripel 296, 302 und 313 nm. Die verwendeten Strahler haben zwischen 315 und 340 nm jedoch eine deutliche Lücke in der Linienbesetzung, so daß in diesem Bereich so gut wie keine Strahlung erzeugt wird.

Da die für die Filter eingesetzten Gläser jedoch erst für UV-Licht mit Wellenlängen größer 300 nm durchlässig werden, ergibt sich die Tatsache, daß bei den verwendeten Quecksilberbrennern im wesentlichen nur die Strahlung mit 313 nm Wellenlänge für die Bräunung zur Verfügung steht.

Der Erfindung liegt die Aufgabe zugrunde, eine Strahlenquelle mit einem nachgeschalteten Filter zur Absorption unerwünschter Strahlung zu schaffen, mit dem eine optimale Einstellung des Bräunungsgerätes möglich ist und ungeeignete Filter nach ihrer Herstellung aussortiert werden können.

Diese Aufgabe wird dadurch gelöst, daß die Transmissionsrate des Filters für eine Strahlung mit einer Wellenlänge von 313 nm bestimmt wurde und deren Wert zur Kennzeichnung des Filters angegeben ist, wobei als Strahlenquelle ein Quecksilberhochdruckbrenner verwendet wird. Die Aufgabe wird weiterhin gelöst durch ein Verfahren zur Bestimmung der Transmissionsrate für eine Strahlung mit einer Wellenlänge von 313 nm sowie durch ein Bräunungsverfahren, dessen die Bräunung beeinflussenden Parameter in Abhängigkeit von der Transmissionsrate eines speziellen Filters für eine Strahlung mit 313 nm Wellenlänge eingestellt bzw. geregelt werden.

Bei den erfindungsgemäßen Filtern wird zunächst die Transmissionsrate für eine Strahlung mit einer Wellenlänge von 313 nm bestimmt. Dies erfolgt beispielsweise gemäß Anspruch 9, indem nämlich zunächst die Strahlungsintensität einer Strahlenquelle für die emittierte Strahlung mit einer Wellenlänge von 313 nm mittels eines auf diese Wellenlänge eingestellten Emissionsmeßgerätes bei einer vorgegebenen Leistung der Strahlenquelle gemessen wird. Der entsprechende Meßwert wird gleich 100 % gesetzt.

Anschließend wird die entsprechende Strahlungsintensität nach Durchgang der Strahlung durch den jeweils zu untersuchenden Filter gemessen. Durch den prozentualen Vergleich der beiden Meßwerte läßt sich nicht nur die Eignung des Glasfilters zum Einsatz in Bräunungsgeräten feststellen; durch die für jeden Filter charakteristische Transmissionsrate können auch die Bräunungsgeräte optimal eingestellt werden. Bei Kenntnis der Transmissionsrate für eine Strahlung von 313 nm können beispielsweise zu diesem Zwecke bestimmte Diagramme oder Kurven angefertigt werden, aus denen die jeweils optimale Einstellung der Bräunungsgeräte hervorgeht.

Es ist außerdem zweckmäßig, wenn der Wert der entsprechenden Transmissionsrate direkt zur Kennzeichnung des Filters auf diesem angegeben wird. Dadurch werden Verwechslungen ausgeschlossen.

Wie bereits erwähnt, ist es zweckmäßig, als Strahlenquelle eine eisendotierte Quecksilberhochdrucklampe zu verwenden, da bei dieser die für die Bräunung verantwortlichen UV-Strahlen verstärkt auftreten.

Der erfindungsgemäße Filter besteht vorzugsweise aus gefärbtem Glas, das bei der Herstellung in der Glasschmelze so eingestellt wird, daß nur Strahlung oberhalb einer Wellenlänge von 300 nm, vorzugsweise von etwa 310 nm, transmittiert wird.

Dadurch wird der energiereiche kurzwellige Bereich des UVB-Lichtes weitgehend ausgeschaltet, so daß Schädigungen der Haut bei angepaßten Bräunungszeiten sicher vermieden werden können.

Desweiteren ist es zweckmäßig, die Filter so einzustellen, daß ihr Transmissionsbereich sich bei steigender Temperatur zu größeren Wellenlängen hin verschiebt. Dadurch ist nämlich die Möglichkeit gegeben, die Strahlungsintensität durch unterschiedliche Filtertemperaturen zu variieren. Beispielsweise kann der Filter stärker gekühlt werden, wenn etwa bei einem dunklen Hauttyp eine stärkere Strahlung notwendig ist. Bei einem hellhäutigen Menschen kann dagegen die Kühlung gedrosselt werden, so daß die Temperatur des Filters steigt und aufgrund der Verschiebung des Transmissionsbereiches zu langwelligeren Strahlungsanteilen die Intensität der 313 nm-Strahlung abgemindert wird.

Auf diese Weise läßt sich bei der Kenntnis der Transmissionsrate des Filters die Strahlung mit einer Wellenlänge von 313 nm eine außerordentlich präzise Einstellung bzw. Regelung eines Bräunungsgerätes vornehmen, wobei die Bräunungswirkung der UV-Strahlenquelle verstärkt und die unangenehmen oder schädlichen Einwirkungen auf den Benutzer minimiert sind.

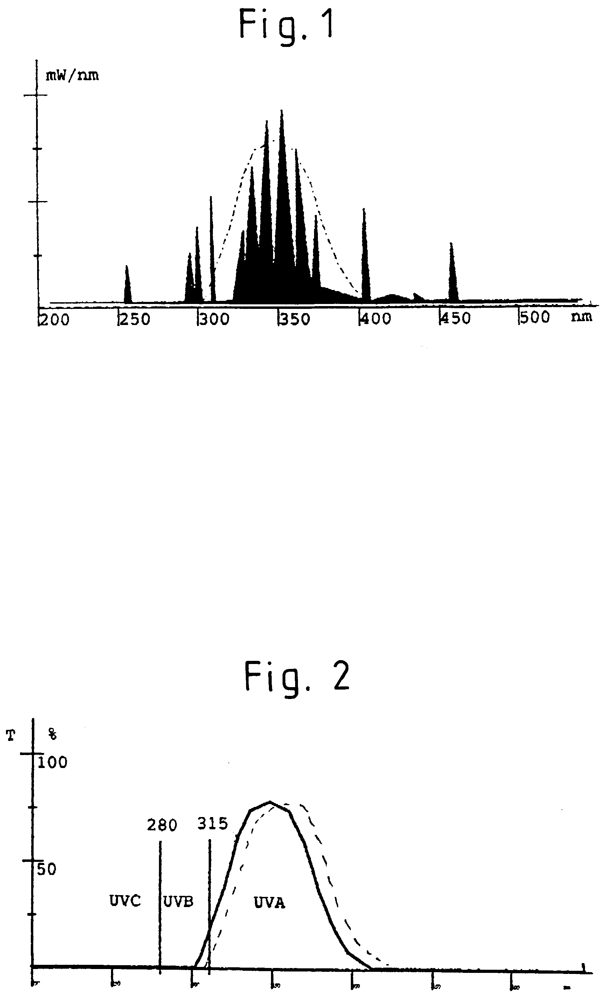

In der zum Verständnis der Erfindung beigefügten Zeichnungs-figur 1 ist eine typische Emissionskurve eines eisendotierten Quecksilberhochdruckstrahlers wiedergegeben, während die Zeichnungsfigur 2 die Transmissionskurve eines erfindungsgemäßen Filters darstellt.

Das in Fig. 1 dargestellte Diagramm zeigt die Strahlungsemissionen eines eisendotierten Quecksilberhochdruckstrahlers. Hierbei ist zu beachten, daß im Bereich des UVB-Lichtes, also zwischen 280 und 315 nm Wellenlänge, lediglich eine Lichtlinie ausprägt, nämlich die Linie bei 313 nm. Zwischen dieser Linie und der nächsten Linie bei ca. 320 nm, befindet sich eine breite Strahlungslücke.

Die mit gestrichelten Linien in das Diagramm eingezeichnete Glockenkurve stellt die Transmissionskurve eines erfindungsgemäßen Filters dar.

Wie besser aus Fig. 2 hervorgeht, öffnet das Filterglas bei einer Wellenlänge von knapp über 300 nm, erreicht zwischen 355 und 370 nm ein Maximum und schließt oberhalb von etwa 420 nm Wellenlänge. Es wird deutlich, daß nur die Wellenlänge bei 313 nm als bräunungsaktiver Bestandteil des UVB-Lichtes vom Filterglas durchgelassen wird. Die Kenntnis dieser Transmissionsrate ist daher entscheidend für die richtige Einstellung eines Bräunungsgerätes.

Wie ebenfalls in Fig. 2 dargestellt, weist der erfindungsgemäße Filter eine Verschiebung seines Transmissionsbereiches zu langwelligerer Strahlung bei Erwärmung auf. Dieser Sachverhalt wird durch die gestrichelte Kurve in Fig. 2 veranschaulicht. Die Transmissionsrate bei einer Erhöhung der Filtertemperatur kann für die 313-Linie stark abgesenkt werden, wodurch sich exakte Einstellungsmöglichkeiten der bräunungsaktiven Bestandteile bzw. des Bräunungsgerätes ergeben.

Die erfindungsgemäßen Filtergläser liegen bzgl. ihrer Dicke zwischen einem und 6 mm, vorzugsweise jedoch zwischen 2,5 und 4 mm, so daß sie hinreichend mechanisch belastbar sind, ohne ein zu großes Gewicht aufzuweisen.

## Patentansprüche

1. Strahlenquelle zur Erzeugung einer insbesondere für kosmetische und/oder medizinische Zwecke verwendbaren Strahlung, mit einem nachgeschalteten Filter zur Absorption oder Abschwächung ungeeigneter oder unerwünschter Strahlung, **dadurch gekennzeichnet,** daß die Transmissionsrate des Filters für eine Strahlung mit einer Wellenlänge von 313 nm bestimmt wurde, deren Wert zur Kennzeichnung des Filters angegeben ist, und als Strahlenquelle ein Quecksilberhochdruckstrahler verwendet wird.

2. Strahlenquelle nach Anspruch 1, **dadurch gekennzeichnet,** daß der Quecksilberhochdruckstrahler mit Eisen dotiert ist.

3. Strahlenquelle nach einem der Ansprüche 1 oder 2, **dadurch gekennzeichnet,** daß der Filter aus Glas besteht.

4. Strahlenquelle nach einem der Ansprüche 1 bis 3, **dadurch gekennzeichnet,** daß der Filter nur Strahlung im UV-Bereich mit einer Wellenlänge oberhalb 300 nm transmittiert.

5. Strahlenquelle nach einem der Ansprüche 1 bis 4, **dadurch gekennzeichnet,** daß der Filter nur Strahlung im UV-Bereich mit einer Wellenlänge oberhalb 310 nm transmittiert.

6. Strahlenquelle nach einem der Ansprüche 1 bis 5, **dadurch gekennzeichnet,** daß der Filter seinen Transmissionsbereich bei steigender Temperatur zu größeren Wellenlängen hin verschiebt.

7. Strahlenquelle nach einem der Ansprüche 1 bis 6, **dadurch gekennzeichnet,** daß die Dicke des Filters zwischen 1 und 6 mm, vorzugsweise zwischen 2,5 und 4 mm liegt.

8. Verfahren zur Bestimmung der Transmissionsrate eines Filters für eine Strahlenquelle gemäß dem Oberbegriff des Anspruchs 1 zur Festlegung der Bräunungswirkung des Strahlenquelle-Filter-Systems, **gekennzeichnet durch** folgende Verfahrensschritte:
a) Messung der Strahlungsintensität einer Strahlenquelle für die emittierte Strahlung mit einer Wellenlänge von 313 nm mittels eines auf 313 nm eingestellten Emissionsmeßgerätes bei einer vorgegebenen Leistung der Strahlenquelle,
b) Messung der Strahlungsintensität gemäß a) mit nachgeschaltetem Filter,
c) Berechnung der Transmissionsrate des Filters für die Wellenlänge von 313 nm bei der vorgegebenen Leistung der Strahlenquelle durch Vergleich der gemessenen Werte.

9. Bräunungsverfahren für Personen unter Verwendung einer UV-Licht-erzeugenden Strahlungsquelle, vorzugsweise eine Quecksilberhochdrucklampe, der ein Filter zur Absorption oder Abschwächung ungeeigneter oder unerwünschter Strahlung nachgeschaltet ist, **dadurch gekennzeichnet,** daß die Transmissionsrate des Filters für eine Strahlung mit einer Wellenlänge von 313 nm bestimmt wird und die die Bräunung beeinflussenden Parameter wie Behandlungsdauer, Leistung der Strahlungsquelle, Tempertur des Filters, Abstand zur behandelten Person in Abhängigkeit der ermittelten Transmissionsrate geregelt bzw. eingestellt wird.
